# EUROPEAN PATENT APPLICATION

(11) **EP 4 656 964 A1**
(43) Date of publication of application: **03.12.2025**
(21) Application number: 24750224.8
(22) Date of filing: 29.01.2024
(51) Int. Cl.: F24F 11/70, A61B 5/11, A61B 5/16, F24F 11/63, F24F 11/89, F24F 120/00

(54) **ENVIRONMENT CONTROL DEVICE**

(30) Priority: 31.01.2023 JP 2023013101
(71) Applicant: Daikin Industries, Ltd., Osaka-shi, Osaka 530-0001 (JP)
(72) Inventor: HATA, Masataka, Osaka-shi, Osaka 530-0001 (JP); NISHINO, Atsushi, Osaka-shi, Osaka 530-0001 (JP); HASHIMOTO, Satoshi, Osaka-shi, Osaka 530-0001 (JP)
(74) Representative: Hoffmann Eitle
(86) International application number: PCT/JP2024/002700
(87) International publication number: WO 2024/162275

(57) **Abstract**

An environment control device including a control unit (100) used to control an environment adjustment portion (A) that controls a thermal environment of a space (I). The control unit (100) is configured to estimate a sensation indicating comfort or discomfort of a target person (T) based on sensation information about the target person (T) in the space (I), determine, based on an appearance frequency of each sensation corresponding to a thermal index value as a value of thermal index, a range of the thermal index value indicating comfort or discomfort as a sensation region, and set a target value of thermal index in the space (I) based on the sensation region.

## Description

### TECHNICAL FIELD

The present disclosure relates to an environment control device, an environment adjustment device, an air conditioner, a control method, and a program.

### BACKGROUND ART

The air conditioner of Patent Document 1 estimates a sensation of a target person (T) based on the amount of change in his/her body surface temperature measured by an infrared ray sensor, and conditions air by using the estimated sensation data.

### CITATION LIST

### PATENT DOCUMENT

Patent Document 1: Japanese Unexamined Patent Publication No. 2018-202103

### SUMMARY OF THE INVENTION

### TECHNICAL PROBLEM

The air conditioner of Patent Document 1 estimates a sensation with the amount of change in the body surface temperature and the sensation being linked together, and thus the estimated sensation may be a sensation affected by factors other than the thermal environment. If thermal control is performed based on a sensation affected by various factors, it is difficult to provide a thermal environment suitable for a target person.

An object of the present disclosure is to provide a thermal environment suitable for a target person.

### SOLUTION TO THE PROBLEM

A first aspect is directed to
an environment control device including a control unit (100) used to control an environment adjustment portion (A) that controls a thermal environment of a space (I),
the control unit (100) being configured to
   estimate a sensation indicating comfort or discomfort of a target person (T) based on sensation information about the target person (T) in the space (I),
   determine, based on an appearance frequency of each sensation corresponding to a thermal index value as a value of thermal index, a range of the thermal index value indicating comfort or discomfort as a sensation region, and
   set a target value of the thermal index in the space (I) based on the sensation region.
   determine a duration of comfort or discomfort sensation, and
   determine the sensation region by excluding the thermal index value where the duration of the comfort or discomfort sensation is shorter than a predetermined time.

In the first aspect, the thermal index value of the space (I) can be set to the target value suitable for the target person (T). The target value is set based only on the sensation indicating comfort or discomfort of the target person (T) caused by the thermal environment, excluding the sensation derived from causes other than the thermal environment of the target person (T). This improves the reliability of the target value of the thermal index.

A second aspect is an embodiment of the first aspect. In the second aspect, the control unit (100) is further configured to associate the target value with a temporal change or an outdoor air temperature.

In the second aspect, for example, the thermal adaptation of the target person (T) to seasonal changes can be reflected in control of the environment adjustment portion (A). This makes it possible to realize comfort thermal environment suitable for the sensation felt by the target person (T) throughout the year.

A third aspect is an embodiment of the first or second aspect. In the third aspect, the control unit (100) is further configured to limit the sensation region to a comfort region in which the sensation indicates comfort, and set the target value to a value in the comfort region.

In the third aspect, the sensation region is limited to the comfort region. This makes it possible to set the thermal index value at which the target person (T) feels comfortable as a target value.

A fourth aspect is an embodiment of any one of the first to third aspects. In the fourth aspect, the control unit (100) is further configured to determine a duration of comfort or discomfort sensation, and determine the sensation region by excluding the thermal index value where the duration of the comfort or discomfort sensation is shorter than a predetermined time.

In the fourth aspect, the thermal index value corresponding to the sensation which disappears in a relatively short time among the sensations exhibited by the target person (T) in the thermal environment is excluded. By removing such noise, the reliability of the target value of the thermal index can be improved.

A fifth aspect is an embodiment of the third aspect. In the fifth aspect, the control unit (100) is further configured to exclude, from the comfort region, one or more of the thermal index values where the sensation of discomfort appeared.

In the fifth aspect, since a more pure comfort region can be acquired, the reliability of the target value can also be increased. For example, even if there are multiple target persons (T) having different thermal sensations in the space (I), it is possible to prevent the thermal index value corresponding to "uncomfortable" from being adopted as the target value.

A sixth aspect is an embodiment of the third or fifth aspect. In the sixth aspect, the control unit (100) is further configured to set, to the target value, the thermal index value where power consumption of the environment adjustment portion (A) in operation is equal to or lower than a predetermined value in the comfort region.

In the sixth aspect, it is possible to achieve both the comfort of the space (I) and the energy saving of the environment adjustment portion (A).

A seventh aspect is an embodiment of the first or second aspect. In the seventh aspect, the control unit (100) is further configured to set the thermal index value between two comfort regions indicating comfort in the sensation region as a neutral thermal index value indicating thermal neutrality, and determine the target value based on the neutral thermal index value.

In the seventh aspect, the discrepancy between the thermal index value output by the control unit (100) and the sensation exhibited by the target person (T) can be reduced.

An eighth aspect of the present disclosure is an embodiment of the seventh aspect. In the eighth aspect, the control unit (100) is further configured to determine a duration of the sensation of comfort, and determine the two comfort regions by excluding the thermal index value where the duration is shorter than a predetermined time.

In the eighth aspect, the thermal index value corresponding to the comfort sensation which disappears in a relatively short time is excluded from the comfort region. By removing such noise, the reliability of the target value can be improved.

A ninth aspect is an embodiment of the seventh or eighth aspect. In the ninth aspect, the thermal index is a predicted mean vote (PMV), and the control unit (100) is further configured to set the neutral thermal index value as PMV = 0 between the two comfort regions.

In the ninth aspect, the target value can be determined based on the PMV.

A tenth aspect is an embodiment of any one of the seventh to ninth aspects. In the tenth aspect, the thermal index is a predicted mean vote (PMV), and the control unit (100) is further configured to set two discomfort regions indicating discomfort in the sensation region, and set PMV = -0.5 to one of the thermal index values between the comfort region and the discomfort region adjacent to each other, and set PMV = +0.5 to the other thermal index value.

In the tenth aspect, the range of -0.5 < PMV < +0.5 can include two comfort regions. In this manner, the PMV output can be corrected easily based on the PMV. Note that -0.5 < PMV < +0.5 is a range recommended as a comfort region.

An eleventh aspect is an embodiment of any one of the first to tenth aspects. In the eleventh aspect, the control unit (100) is further configured to control the environment adjustment portion (A) based on the target value.

In the eleventh aspect, the space (I) can be made into a thermal environment suitable for the target person (T) based on the target value.

A twelfth aspect is an embodiment of any one of the first to eleventh aspects. In the twelfth aspect, the control unit (100) is communicatively connected to a predetermined communication device (80) configured to display an operation screen (81) that allows setting of the thermal index value, and configured to transmit setting information indicating the target value to the communication device (80) and receive information indicating the thermal index value set based on the setting information.

Int twelfth aspect, the target person (T) can grasp the target value by the communication device (80). The target person (T) can control the thermal environment according to his/her preference. This improves the convenience of the environment control device (E). The target person (T) can grasp the thermal state of the space (I) before and after the control, and thus can actually feel a change in thermal environment.

A thirteenth aspect is directed to an environment adjustment device including the environment adjustment portion (A) and the control unit (100), of any one of the first to twelfth aspects.

A fourteenth aspect is directed to an air conditioner including the environment adjustment portion (A) and the control unit (100), of any one of the first to twelfth aspects. The environment adjustment portion (A) is an air conditioning portion (A) configured to condition air in the space (I).

A fifteenth aspect is directed to a control method including: estimating a sensation indicating comfort or discomfort of a target person (T) based on sensation information about a target person (T) in a space (I); determining, based on an appearance frequency of each sensation corresponding to a thermal index value as a value of thermal index, a range of the thermal index value indicating comfort or discomfort as a sensation region; and setting a target value of the thermal index in the space (I) based on the sensation region.

A sixteenth aspect is directed to a program for causing a computer to execute: estimating a sensation indicating comfort or discomfort of a target person (T) based on sensation information about a target person (T) in a space (I); determining, based on an appearance frequency of each sensation corresponding to a thermal index value as a value of thermal index, a range of the thermal index value indicating comfort or discomfort as a sensation region; and setting a target value of the thermal index in the space (I) based on the sensation region.

### BRIEF DESCRIPTION OF THE DRAWINGS

[FIG. 1] FIG. 1 is a schematic configuration diagram of an air conditioner according to an embodiment.
[FIG. 2] FIG. 2 is a piping system diagram of the air conditioner.
[FIG. 3] FIG. 3 is a configuration diagram illustrating an internal structure of an indoor unit of the air conditioner.
[FIG. 4] FIG. 4 is a front view of the indoor unit of the air conditioner.
[FIG. 5] FIG. 5 is a block diagram illustrating main devices of the air conditioner.
[FIG. 6] FIG. 6 is a table showing a relationship between PMV and thermal sensation.
[FIG. 7] FIG. 7 is a flowchart showing an overall flow of setting a first target PMV.
[FIG. 8] FIG. 8 is a table showing data (first thermal data) in which sensation, PMV, and duration are associated with each other.
[FIG. 9] FIG. 9 is a graph showing an example of frequency distribution (first distribution data) indicating a relationship between PMV and a frequency of appearing sensation. FIG. 9A is the first distribution data in which the sensation is comfort, and FIG. 9B is the second distribution data in which the sensation is discomfort.
[FIG. 10] FIG. 10 is a table showing an example of data (second thermal data) in which each month and first target PMV are associated with each other.
[FIG. 11] FIG. 11 is a flowchart of setting the first target PMV.
[FIG. 12] FIG. 12 is a diagram for explaining a flow of selecting the first target PMV.
[FIG. 13] FIG. 13 is a flowchart showing an overall flow of setting the second target PMV.
[FIG. 14] FIG. 14 is a graph showing an example of second distribution data (frequency distribution showing a relationship between PMV (thermal index value) and frequency of each appearing sensation throughout the year). FIG. 14A is the second distribution data before correction, and FIG. 14B is the corrected second distribution data.
[FIG. 15] FIG. 15 is a diagram showing an example of operation screen displayed on an information terminal.

### DESCRIPTION OF EMBODIMENTS

Embodiments of the present invention will be described in detail below with reference to the drawings. The following embodiments are merely exemplary ones in nature, and are not intended to limit the scope, application, or uses of the invention. Features of the embodiments, variations, and other examples described below can be combined or partially substituted within the range where the present invention can be embodied.

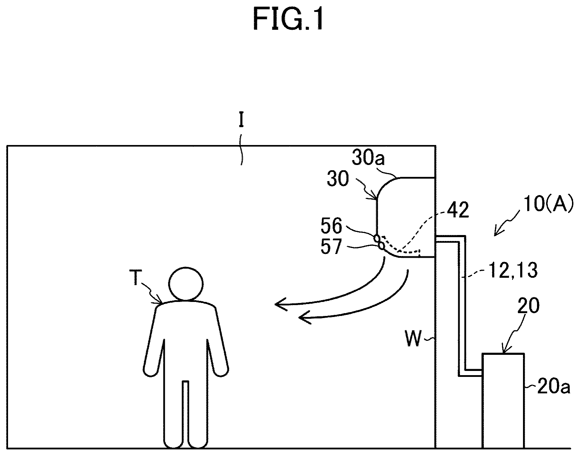

An environment control device (E) of the present disclosure is applied to an air conditioner (10). The air conditioner (10) is an example of an environment adjustment device. As illustrated in FIG. 1, the air conditioner (10) provides an environmental stimulus to a target person (T) in an indoor space (I), which is a target space. The air conditioner (10) adjusts an environment of the indoor space (I). The air conditioner (10) conditions air in the indoor space (I). The air conditioner (10) of this embodiment controls the temperature of air in the indoor space (I). The indoor space (I) is an example of the space (I) of the present disclosure.

### (1) Configuration of Air Conditioner

### (1-1) General Configuration

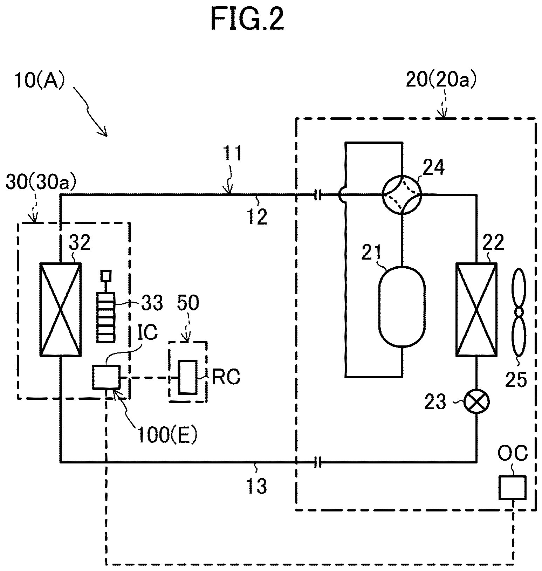

As illustrated in FIGS. 1 and 2, the air conditioner (10) includes an outdoor unit (20), an indoor unit (30), a first connection pipe (12), and a second connection pipe (13). The air conditioner (10) is a pair-type air conditioner including one outdoor unit (20) and one indoor unit (30). The first connection pipe (12) is a gas connection pipe, and the second connection pipe (13) is a liquid connection pipe. The outdoor unit (20) and the indoor unit (30) are connected to each other via the first connection pipe (12) and the second connection pipe (13) to constitute a refrigerant circuit (11). The refrigerant circuit (11) circulates the refrigerant therethrough to perform a refrigeration cycle. The refrigerant is, for example, difluoromethane. The air conditioner (10) includes an environment adjustment portion (A) and a control unit (100), which will be described later.

### (1-2) Outdoor Unit

The outdoor unit (20) is installed outdoors. The outdoor unit (20) includes an outdoor casing (20a), a compressor (21), an outdoor heat exchanger (22), an expansion valve (23), a four-way switching valve (24), and an outdoor fan (25). The outdoor casing (20a) houses the compressor (21), the outdoor heat exchanger (22), the expansion valve (23), the four-way switching valve (24), and the outdoor fan (25).

The compressor (21) is, for example, a rotary compressor of an oscillating piston type, a rotary type, or a scroll type. The outdoor heat exchanger (22) is a fin-and-tube heat exchanger. The four-way switching valve (24) switches between a first state (the state indicated by the solid curves in FIG. 2) and a second state (the state indicated by the broken curves in FIG. 2). The four-way switching valve (24) in the first state makes a discharge portion of the compressor (21) and a gas end of the outdoor heat exchanger (22) communicate with each other, and makes a suction portion of the compressor (21) and the first connection pipe (12) communicate with each other. The four-way switching valve (24) in the second state makes the discharge portion of the compressor (21) and the first connection pipe (12) communicate with each other, and makes the suction portion of the compressor (21) and the gas end of the outdoor heat exchanger (22) communicate with each other. The outdoor fan (25) is a propeller fan.

### (1-3) Indoor Unit

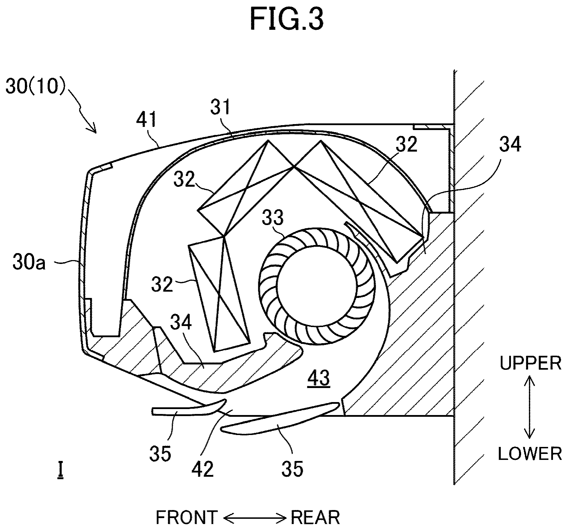

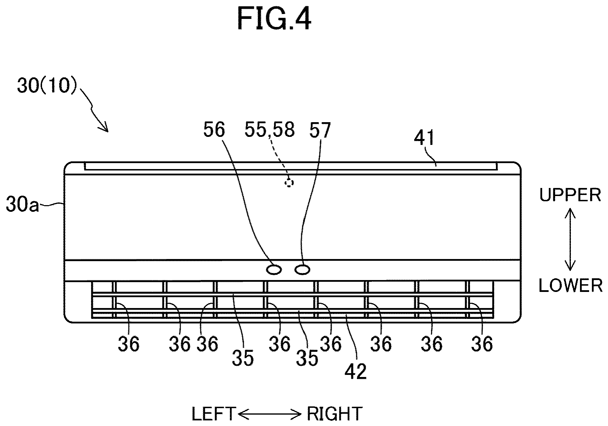

The indoor unit (30) illustrated in FIGS. 3 and 4 is installed in the indoor space (I). The indoor unit (30) is a wall-mounted unit installed on a wall (W) of the indoor space (I). The indoor unit (30) houses an indoor casing (30a), an air filter (31), an indoor heat exchanger (32), an indoor fan (33), a drain pan (34), first flaps (35), and second flaps (36).

The indoor casing (30a) is formed in a hollow shape that is long in the left-right direction. The indoor casing (30a) houses the air filter (31), the indoor heat exchanger (32), the indoor fan (33), the drain pan (34), the first flaps (35), and the second flaps (36). The indoor casing (30a) has an inlet (41) and an outlet (42). The inlet (41) is formed in an upper portion of the indoor casing (30a). The inlet (41) is an opening through which air is sucked into the indoor space (I). The inlet (41) extends in the longitudinal direction (left-right direction) of the indoor casing (30a). The outlet (42) is formed near the front side in a lower portion of the indoor casing (30a). The outlet (42) extends in the longitudinal direction of the indoor casing (30a). The indoor casing (30a) includes therein an air passage (43) from the inlet (41) to the outlet (42).

The air filter (31) is disposed upstream of the indoor heat exchanger (32) in the air passage (43). The air filter (31) is a mesh member formed along the inlet (41). The air filter (31) catches dust in intake air sucked through the inlet (41).

The indoor heat exchanger (32) is disposed upstream of the indoor fan (33) in the air passage (43). The indoor heat exchanger (32) is a fin-and-tube heat exchanger. The indoor heat exchanger (32) allows heat exchange between the refrigerant flowing therethrough and air transferred by the indoor fan (33).

The indoor fan (33) is an example of a fan. The indoor fan (33) is a cross-flow fan. The indoor fan (33) extends in the longitudinal direction of the indoor casing (30a). The indoor fan (33) is rotationally driven by a fan motor (33a). The indoor fan (33) transfers air in the air passage (43). When the indoor fan (33) is driven, air in the indoor space (I) is sucked into and flows through the air passage (43). At the same time, the air in the air passage (43) is blown out through the outlet (42). The indoor fan (33) is configured to adjust the volume of blown air supplied to the indoor space (I) through the outlet (42). The number of revolutions of the fan motor (33a) is controlled to control the volume of blown air.

The drain pan (34) is disposed below the indoor heat exchanger (32). The drain pan (34) is a tray which receives water generated in the indoor casing (30a). The drain pan (34) receives condensation water generated on the surface of the indoor heat exchanger (32).

The first flaps (35) and the second flaps (36) constitute an airflow direction adjustment portion that adjusts the airflow direction of blown air. The indoor unit (30) includes two first flaps (35) and eight second flaps (36), but these numbers are mere examples. The first flaps (35) adjust the up-and-down direction of the blown air. The second flaps (36) adjust the left-right direction of the blown air. The two first flaps (35) are arranged in the up-and-down direction. The first flaps (35) extend in the longitudinal direction of the indoor casing (30a). The first flaps (35) are driven by a first flap motor (35a). The multiple second flaps (36) are arranged in the longitudinal direction of the indoor casing (30a). The second flaps (36) extend along the up-and-down direction. The second flaps (36) are driven by a second flap motor (36a).

### (1-4) Remote Controller

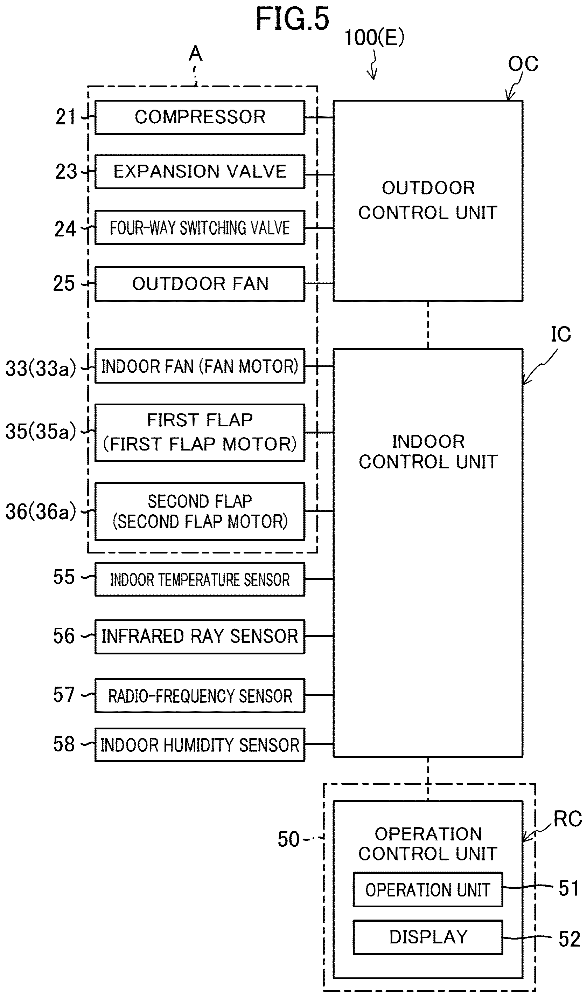

As illustrated in FIGS. 2 and 5, the air conditioner (10) includes a remote controller (50). The remote controller (50) includes an operation unit (51) and a display (52). The operation unit (51) allows the user to input various instructions to the air conditioner (10). The operation unit (51) is a button, a switch, or a touch panel. The instructions referred to herein include switching the air conditioner (10) between ON and OFF, selecting the operating mode of the air conditioner (10), and changing the set temperature of the indoor space (I). The display (52) displays information on the state and the operation of the air conditioner (10). This information includes the operating mode and the set temperature of the air conditioner (10).

### (1-5) Sensor

The air conditioner (10) includes multiple sensors. The multiple sensors include an indoor temperature sensor (55), an infrared ray sensor (56), a radio-frequency sensor (57), and an indoor humidity sensor (58). The indoor temperature sensor (55) and the indoor humidity sensor (58) are disposed near the inlet (41). As illustrated in FIG. 4, the infrared ray sensor (56) and the radio-frequency sensor (57) are disposed on the front surface of the indoor casing (30a). The infrared ray sensor (56) and the radio-frequency sensor (57) are disposed at an intermediate position in the longitudinal direction (left-right direction) on the front surface of the indoor casing (30a).

The indoor temperature sensor (55) detects the temperature of air in the indoor space (I). The indoor temperature sensor (55) detects the temperature of air sucked into the inlet (41).

The indoor humidity sensor (58) detects the humidity of air in the indoor space (I). The indoor humidity sensor (58) detects the humidity of air sucked into the inlet (41).

The infrared ray sensor (56) detects the temperature distribution of air in the indoor space (I) and the surface temperature of a person in the indoor space (I). The infrared ray sensor (56) is used to divide the indoor space (I) into multiple two-dimensional sections and acquire data of the temperatures of the sections.

The radio-frequency sensor (57) is a sensor for acquiring sensation information about the target person (T). The radio-frequency sensor (57) is a vital sensor that detects biological signals of the target person (T) by using microwaves. The radio-frequency sensor (57) is a non-contact vital sensor. In other words, the radio-frequency sensor (57) can detect biological signals of the target person (T) without contact with the target person (T). The biological signals include signals derived from respiration, heartbeat, pulse waves, brain waves, body movement, and the like of the target person (T).

### (1-6) Control Unit

The control unit (100) constitutes an environment control device (E) that controls an air conditioner (10). Strictly speaking, the control unit (100) controls an air conditioning portion (A). The air conditioning portion (A) is a mechanical element required to perform air conditioning of the indoor space (I). The air conditioning portion (A) constitutes an environment adjustment portion (A) that controls a thermal environment of the indoor space (I). The control unit (100) is used to control the environment adjustment portion (A).

As illustrated in FIG. 5, the control unit (100) includes an indoor control unit (IC), an outdoor control unit (OC), and an operation control unit (RC). The indoor control unit (IC), the outdoor control unit (OC), and the operation control unit (RC) are configured to communicate with each other in a wired or wireless manner. The indoor control unit (IC), the outdoor control unit (OC), and the operation control unit (RC) each include a micro control unit (MCU), an electric circuit, and an electronic circuit. The MCU includes a central processing unit (CPU), a memory, and a communications interface. The memory stores various programs that are executed by the CPU.

The outdoor control unit (OC) is provided for the outdoor unit (20). The outdoor control unit (OC) is disposed inside the outdoor casing (20a). The outdoor control unit (OC) controls the compressor (21), the expansion valve (23), the four-way switching valve (24), and the outdoor fan (25). Strictly speaking, the outdoor control unit (OC) controls start and stop of the compressor (21), the number of revolutions of the compressor (21), the opening degree of the expansion valve (23), the state of the four-way switching valve (24), start and stop of the outdoor fan (25), and the number of revolutions of the outdoor fan (25).

The indoor control unit (IC) is provided for the indoor unit (30). The outdoor control unit (OC) is disposed inside the indoor casing (30a). The indoor control unit (IC) controls the indoor fan (33). Specifically, the indoor control unit (IC) controls start and stop of the indoor fan (33) and the number of revolutions of the fan motor (33a) of the indoor fan (33). The indoor control unit (IC) controls the first flaps (35) and the second flaps (36). Specifically, the indoor control unit (IC) controls the first flap motor (35a) and the second flap motor (36a) so as to adjust the angular positions of the first flaps (35) and the second flaps (36).

The detection signals detected by the indoor temperature sensor (55), the infrared ray sensor (56), and the radio-frequency sensor (57) are input to the indoor control unit (IC).

The operation control unit (RC) transmits, to the indoor control unit (IC), a command for the operating mode and the set temperature input by the user using the operation unit (51). This command is transmitted from the indoor control unit (IC) to the outdoor control unit (OC).

### (2) Basic Operation

The air conditioner (10) performs the cooling operation and the heating operation.

### (2-1) Cooling Operation

The cooling operation is an operation for cooling air in the indoor space (I) so that the air in the indoor space (I) is brought closer to the set temperature (target temperature). In the cooling operation, the four-way switching valve (24) is switched to the first state. The refrigerant that has compressed in the compressor (21) dissipates heat in the outdoor heat exchanger (22) and is then decompressed in the expansion valve (23). The refrigerant that has been decompressed evaporates in the indoor heat exchanger (32). The air that has cooled in the indoor heat exchanger (32) is supplied to the indoor space (I). The refrigerant that has evaporated in the indoor heat exchanger (32) is sucked into the compressor (21).

### (2-2) Heating Operation

The heating operation is an operation for heating air in the indoor space (I) so that the air in the indoor space (I) is brought closer to the set temperature (target temperature). In the heating operation, the four-way switching valve (24) is switched to the second state. In the heating operation, the refrigerant that has compressed in the compressor (21) dissipates heat in the indoor heat exchanger (32), and is then decompressed in the expansion valve (23). The air that has heated in the indoor heat exchanger (32) is supplied to the indoor space (I). The refrigerant that has been decompressed evaporates in the outdoor heat exchanger (22), and then is sucked into the compressor (21).

### (3) Comfort Operation

The air conditioner (10) performs a comfort operation for controlling the air conditioner (10) so as to establish a thermal environment in the indoor space (I) which is suitable for the target person (T). In order to perform the comfort operation, the control unit (100) sets a target value of a thermal index based on the data indicating the estimated sensation of the target person (T) and the thermal index value for the indoor space (I) corresponding to the sensation. The control unit (100) controls the air conditioner (10) so that the value indicating the thermal index for the indoor space (I) reaches the target value.

The thermal index of this embodiment is a predicted mean vote (PMV), which is an index indicating the thermal environment. The PMV is an index determined based on four physical variables of the temperature, mean radiant temperature, the relative humidity, and the airflow, and two personal variables of the amount of clothing and the amount of activity. A value indicating the thermal index may also be referred to as a thermal index value or merely PMV. The target value of the thermal index may also be referred to as target PMV.

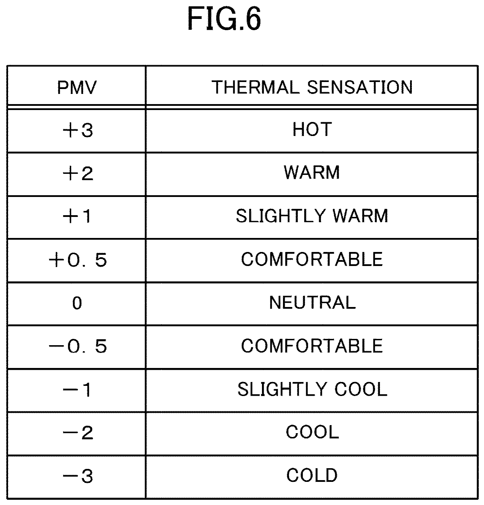

As illustrated in FIG. 6, in the thermal index value (PMV), the comfort/discomfort degrees are classified in the range of 3 ≤ PMV ≤ +3. The control unit (100) of this embodiment determines the PMV of the indoor space (I) based on the ambient temperature, the relative humidity, the airflow, and the amount of activity of the target person (T).

The target PMV includes two types of target PMVs (a first target PMV and a second target PMV) that are set by different methods. The comfort operation includes a first operating mode for operating the air conditioner (10) based on the first target PMV, and a second operating mode for operating the air conditioner (10) based on the second target PMV. The first operating mode and second operating mode are selected by the target person (T). The first target PMV and second target PMV will be described in detail below.

### (4) First Target PMV

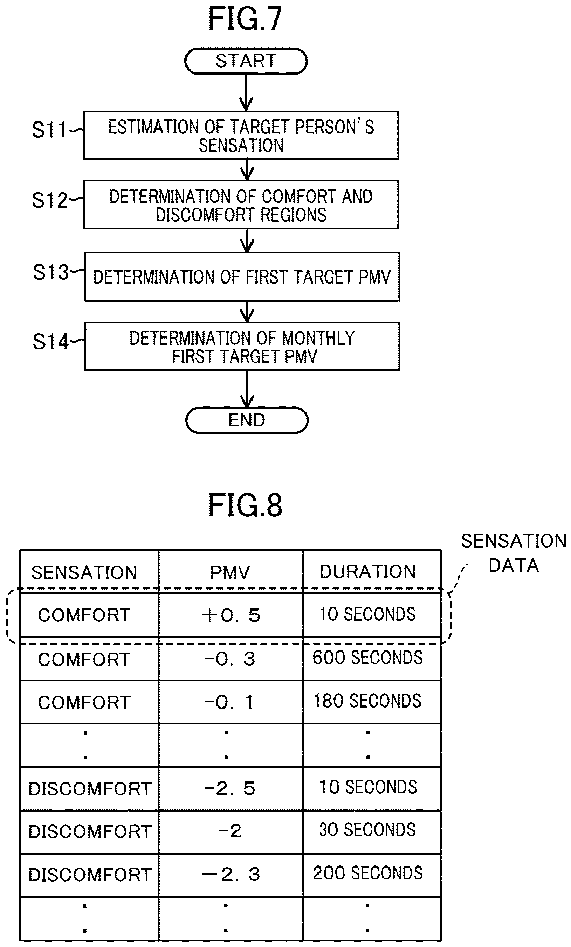

The control unit (100) sets the first target PMV based on the thermal index value (PMV) corresponding to the "comfort" sensation exhibited by the target person (T). The operation of setting the first target PMV by the control unit (100) will be described below with reference to FIGS. 7 to 10.

In Step S11, the control unit (100) estimates a sensation indicating comfort or discomfort of the target person (T) based on the sensation information about the target person (T) in the indoor space (I). The sensation information is biological signals detected by the radio-frequency sensor (57). The control unit (100) estimates the sensation of the target person (T) based on comfort/discomfort valence acquired from the biological signals of the target person (T).

The comfort/discomfort valence can be estimated based on the index indicating the state of autonomic nerve. The parameters of the state of autonomic nerve include autonomic nervous balance (LF/HF) and autonomic nervous activity (SDNN). These parameters can be all acquired based on heartbeat components extracted from the biological signals detected by the radio-frequency sensor (57).

The "LF/HF" indicates the balance between the sympathetic nerve and the parasympathetic nerve of the target person (T). The sensation estimation unit (60) performs, for example, frequency analysis of heartbeat intervals to determine a low-frequency component (LF) in a range between 0.05 Hz to 0.20 Hz and a high-frequency component (HF) of 0.20 Hz or higher, thereby determining the ratio of these components as LF/HF. The HF is greater when the parasympathetic nerves dominate the sympathetic nerves, and the LF is greater when the sympathetic nerves dominate the parasympathetic nerves. Thus, if the target person (T) feels uncomfortable and is highly stressed, the target person (T) has a higher LF/HF. Conversely, if the target person (T) feels comfortable and is less stressed, the target person (T) has a lower LF/HF.

The SDNN is an index indicating the variation of N-N intervals. The SDNN is, for example, a standard deviation of N-N intervals in five minutes. The SDNN is greater when the parasympathetic nerves dominate the sympathetic nerves, and smaller when the sympathetic nerves dominate the parasympathetic nerves. Thus, if the target person (T) feels uncomfortable and is highly stressed, the target person (T) has a smaller SDNN. Conversely, if the target person (T) feels comfortable and is less stressed, the target person (T) has a greater SDNN.

In Step S12, based on the appearance frequency of the sensations corresponding to the respective PMVs (thermal index values), the control unit (100) determines a range of PMVs indicating comfort as a comfort region and a range of PMVs indicating discomfort as a discomfort region. The comfort region and the discomfort region correspond to a sensation region of the present disclosure. The sensation region will be specifically described below.

As illustrated in FIG. 8, the control unit (100) stores the data (hereinafter referred to as the "sensation data") in which the estimated sensation (comfort or discomfort) of the target person (T), the PMV at the time when the sensation is estimated, and the duration during which the sensation is continued are associated with each other. The control unit (100) continuously accumulates the sensation data for a predetermined period of time (e.g., one year) to acquire first thermal data.

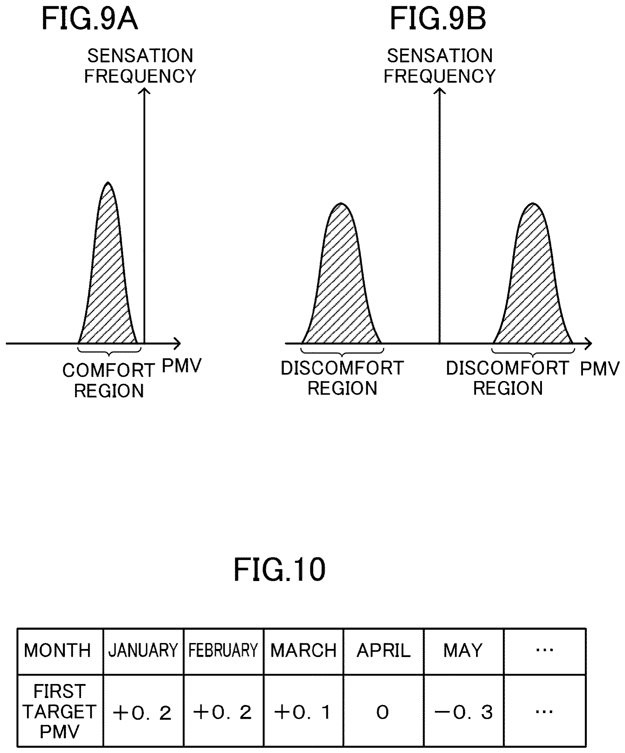

As illustrated in FIG. 9, the control unit (100) creates first distribution data indicating a distribution (frequency distribution) of the appearance frequency of "comfort" and "discomfort" based on the first thermal data. The control unit (100) creates monthly frequency distributions of one year. FIG. 9 is an example of distribution data of a certain month. In this manner, the control unit (100) sets the comfort region and the discomfort region based on the first distribution data.

In Step S13, the control unit (100) limits the sensation region obtained in Step S12 to a comfort region indicating comfort, and sets the PMV (thermal index value) in the comfort region to the first target PMV. In this manner, the control unit (100) sets the target value of the thermal index for the indoor space (I) based on the sensation region.

In Step S14, the control unit (100) determines the first target PMV obtained in Step S13 monthly throughout the year. The control unit (100) acquires second thermal data in which the first target PMV is associated with each month (FIG. 10). In this manner, the control unit (100) associates the target value with the temporal change.

As described above, in the first operating mode, the control unit (100) sets the first target PMV of each month, and controls the air conditioner (10) so that the PMV for the indoor space (I) becomes the first target PMV.

### (4-1) Details of First Target PMV

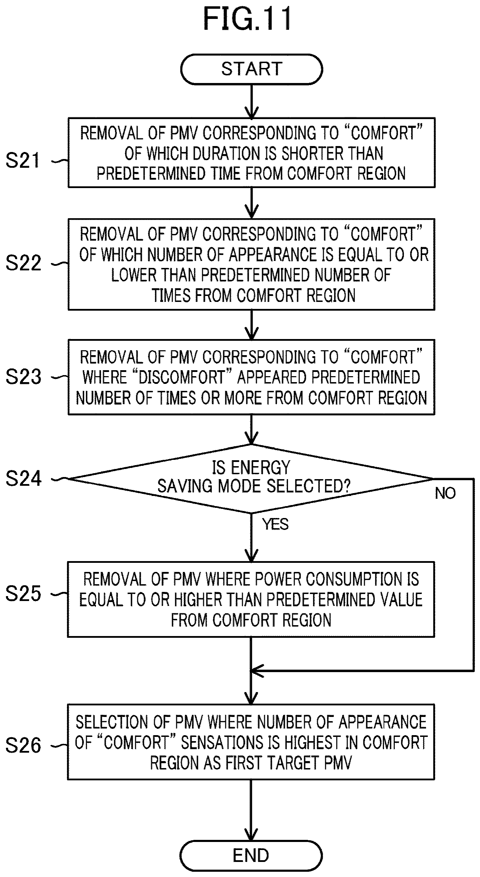

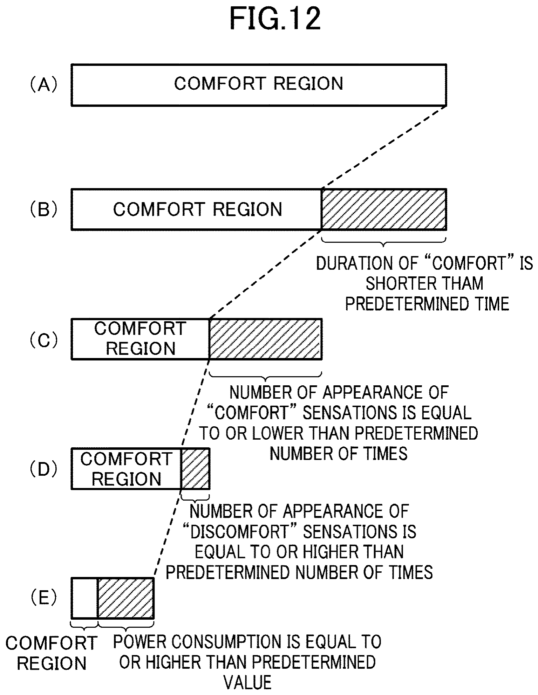

The settings of the first target PMV in Step S13 will be described with reference to FIGS. 11 and 12.

In Step S21, if the duration of "comfort" that appeared is shorter than the predetermined time, the control unit (100) excludes the PMV corresponding to this "comfort" to set the "comfort region." Specifically, the control unit (100) obtains a new comfort region by excluding, from the comfort region obtained in Step S12 ((A) of FIG. 12), the PMV corresponding to "comfort" of which the duration is shorter than the predetermined time ((B) of FIG. 12). The predetermined time may be a relatively short time, for example, 10 seconds. The predetermined time can be determined based on the separation degree between a distribution of durations of target person's (T) comfort sensations caused by the thermal environment and a distribution of durations of target person's (T) comfort sensations not caused by the thermal environment (e.g., conversation, watching TV, and the like). Since the durations of the target person's (T) comfort sensations caused by the thermal environment and the durations of the target person's (T) comfort sensations not caused by the thermal environment overlap each other, a portion where the separation degree between both the distributions is relatively high is extracted to exclude the durations of the comfort sensations not caused by the thermal environment, thereby determining the durations of the comfort sensations caused by the thermal environment. In this manner, the PMV indicating temporal "comfort" sensation is excluded.

In Step S22, the control unit (100) excludes, from the comfort region obtained in Step S21, the PMV where the number of appearance (the appearance frequency) of "comfort" sensations is equal to or lower than the predetermined number of times ((C) of FIG. 12). In this manner, the PMV corresponding to the "comfort" sensation that rarely appears is excluded.

In Step S23, the control unit (100) excludes, from the comfort region obtained in Step S21, one or more PMVs where the discomfort sensations appeared ((D) of FIG. 12). Specifically, in the comfort region, the discomfort sensations may appear in addition to the comfort sensations. In this embodiment, the control unit (100) excludes, from the comfort region obtained in Step S21, the PMV where the discomfort sensations appeared the predetermined number of times or more.

In Step S24, the control unit (100) determines whether the energy saving mode is selected. The energy saving mode is an operation for reducing the power consumption of the air conditioner (10) in operation. The execution of the energy saving mode is selected by the target person (T). If the energy saving mode is selected (YES in Step S24), Step S24 is executed. If the energy saving mode is not selected (NO in Step S24), Step S25 is executed.

In Step S25, the control unit (100) excludes, from the PMVs in the comfort region set in Step S23, the PMV where the power consumption of the air conditioner (10) in operation is equal to or greater than the predetermined value ((E) of FIG. 12).

In Step S26, the control unit (100) determines the PMV where the number of appearance of comfort sensations is highest in the comfort region set in Step S24 as the first target PMV. In this manner, if the energy saving mode is selected in Step S25, the control unit (100) sets the target value where the power consumption of the air conditioner (A) in operation is equal to or lower than the predetermined value in the comfort region.

### (5) Second Target PMV

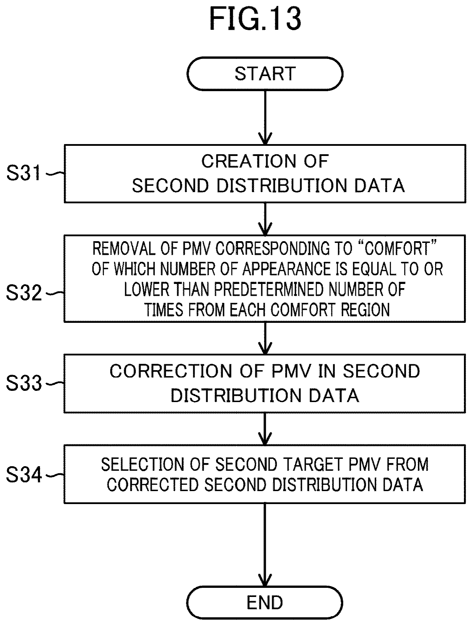

The control unit (100) sets the PMV (thermal index value) between two comfort regions indicating comfort in the sensation region to PMV = 0 indicating thermal neutrality, and determines a second target PMV based on PMV = 0. The PMV=0 corresponds to neutral thermal index value of the present disclosure. The operation of setting the second target PMV by the control unit (100) will be described below with reference to FIGS. 13 to 14.

In Step S31, the control unit (100) creates second distribution data indicating the relationship between the PMV and the appearance frequency of each sensation based on the first thermal data. The first temperature data is created in the same manner as in Step S11 and Step S12. Specifically, the control unit (100) estimates comfort/discomfort sensation of the target person (T) based on the sensation information about the target person (T) in the indoor space (I). Then, based on the appearance frequency of the sensations corresponding to the respective PMVs (thermal index values), the control unit (100) determines a range of PMVs indicating comfort as a comfort region and a range of PMVs indicating discomfort as a discomfort region. The second distribution data is created based on the sensation data for one year, for example. The second distribution data will be described below.

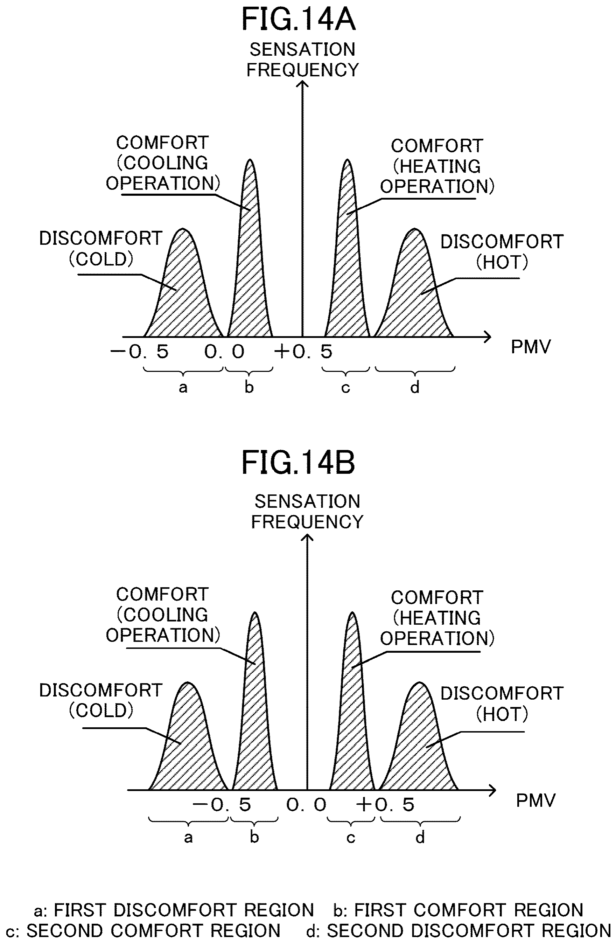

As illustrated in FIG. 14A, in the second distribution data, a first discomfort region in which the target person (T) feels cold appears in a region with the relatively low PMV (region a). A second discomfort region in which the target person (T) feels hot is a region with the relatively high PMV (region d). The first comfort region in which the target person (T) feels comfortable during the cooling operation appears on the side of higher PMV than that in the first discomfort region, so as to be adjacent to the first discomfort region (region b). The second comfort region in which the target person (T) feels comfortable during the heating operation appears on the side of lower PMV than that in the second discomfort region, so as to be adjacent to the second discomfort region (region c).

In Step S32, the control unit (100) excludes PMVs where the durations of "comfort" that appeared is shorter than the predetermined time to determine two comfort regions (the first comfort region and the second comfort region). The predetermined time may be a relatively short, for example, 10 seconds. For the predetermined time, by the method mentioned in Step S21, the durations of comfort sensations not caused by the thermal environment can be excluded, and the durations of the comfort sensations caused by the thermal environment can be determined.

As illustrated in FIG. 14B, in Step S33, the value of PMV is corrected to correspond to each sensation of the target person (T) in which the PMV of the second distribution data appears. Specifically, the control unit (100) sets PMV = 0 between two comfort regions (the first comfort region and the second comfort region). Further, the control unit (100) sets PMV = -0.5 to one of thermal index values between the comfort regions and the discomfort regions adjacent to each other, and sets PMV = +0.5 to the other. In other words, the control unit (100) sets PMV = -0.5 between the first comfort region and the first discomfort region, and sets PMV = +0.5 between the second comfort region and the second discomfort region. In this manner, the PMV (FIG. 14A) of the second distribution data outputted first is corrected, and thus, the corrected second distribution data indicating the PMV corresponding to each sensation of the target person (T) is created (FIG. 14B).

In Step S34, the control unit (100) sets the second target PMV based on the second distribution data corrected in Step S33. The second target PMV may be, for example, PMV = 0. The control unit (100) may associate the second target PMV with the temporal change. For example, during the cooling operation performed in summer, the second target PMV may be set within the range of -0.5 < PMV < 0. During the heating operation performed in winter, the second target PMV may be set within the range of 0 < PMV < +0.5. In this manner, the control unit (100) sets the target value of the thermal index for the indoor space (I) based on the sensation region.

### (5-1) Details of Correction Processing

Next, an example of correction of PMV in Step S33 will be described in detail. The PMV between the first comfort region (PMV with comfort sensation appearing during the cooling operation) and the second comfort region (PMV with comfort sensation appearing during the heating operation) is a thermally neutral condition in which the target person (T) feels neither hot nor cold. Thus, the control unit (100) horizontally moves PMV = 0 by k (k > 0) in the plus direction so that the PMV = 0 is arranged between the first comfort region and the second comfort region.

Further, the control unit (100) multiplies the PMV output value by the correction coefficient α1 so that PMV = +0.5 is arranged between the second comfort region and the second discomfort region. Specifically, when the corrected positive PMV is the first corrected PMV, the control unit (100) performs the calculation according to the first corrected PMV = α1 × (PMV output value + k). The control unit (100) multiplies the PMV output value in the third data by the correction coefficient α2 so that PMV = -0.5 is arranged between the first comfort region and the first discomfort region. Specifically, when the corrected negative PMV is the second corrected PMV, the control unit (100) performs the calculation according to the second corrected PMV = α2 × (PMV output value + k).

### (6) Method of Controlling Comfort Operation

As described above, the control method of the present disclosure includes the processes described in the above sections (3), (4), (4-1), (5), and (5-1), and FIGS. 7 to 14.

Specifically, the control method includes: estimating a sensation indicating comfort or discomfort of a target person (T) based on sensation information about the target person (T) in the indoor space (I); determining, based on an appearance frequency of each sensation corresponding to a thermal index value, a range of the thermal index value indicating comfort or discomfort as a sensation region; and setting a target value of the thermal index for the indoor space (I) based on the sensation region.

The control method of the present disclosure may include processes described in detail in the following variations and other embodiments.

### (7) Program

The storage of the control unit (100) stores a program for causing a computer to execute the control method related to the comfort operation described above. The control method referred to herein includes all the processes described in the above section (6) "Method of Controlling Comfort Operation."

### (8) Advantages of Embodiments

(8-1)
Then, the control unit (100) of the embodiment estimates a sensation indicating comfort or discomfort of a target person (T) based on sensation information about the target person (T) in the indoor space (I), and determines, based on an appearance frequency of each sensation corresponding to PMV (a thermal index value as a value of thermal index), a range of the PMV (thermal index value) indicating comfort or discomfort as a sensation region. The control unit (100) sets, based on the sensation region, the target PMV (the target value of thermal environment) of the indoor space (I).

The appearance frequency of each sensation (comfort and discomfort) corresponding to the PMV of the indoor space (I) is derived from the thermal environment of the indoor space (I). Accordingly, the sensation of the target person (T) derived from other factors than the thermal environment is removed, and the target PMV can be set based on only the comfort or discomfort sensation of the target person (T), caused by the thermal environment, thereby improving the reliability of the target PMV.

In addition, since the target PMV is set based on the sensation exhibited by the target person (T), the thermal environment of the indoor space (I) can be optimized for the target person (T).

In addition, since the sensation of the target person (T) is automatically estimated, there is no need for the target person (T) to report whether he/she feels comfortable or not. For example, by estimating the sensation of the target person (T) in the room every second, the number of sensation data sets that can be acquired increases, and the reliability of the target PMV obtained is further improved.

(8-2)
The control unit (100) of the embodiment associates the target PMV with each month (predetermined period) of one year.

The thermal adaptation of the target person (T) to seasonal changes can be reflected in control of the air conditioner (10). This makes it possible to realize comfort thermal environment suitable for the target person (T) throughout the year.

(8-3)
The control unit of the embodiment limits the sensation region to a comfort region in which the target person (T) exhibits comfort sensation, and sets the target PMV to a value within the comfort region. The first target PMV can be set based on the PMV within the comfort region.

(8-4)
The control unit (100) of the embodiment determines the duration of the comfort or discomfort sensation, and sets the sensation region by excluding the PMV where the duration is shorter than the predetermined time.

In this manner, the sensation data having the PMV corresponding to the sensation which disappears in a relatively short time among the sensations exhibited by the target person (T) with respect to the thermal environment is excluded. By removing such noise, the reliability of the target PMV can be improved.

(8-5)
The control unit (100) of the embodiment excludes one or more PMVs including discomfort sensation in the comfort region.

Although the "discomfort" sensation appears also in the comfort region, the multiple MPVs with a relatively large number of such "discomfort" are excluded to improve the reliability of the comfort region and enhance the reliability of the target PMV.

Such a case where the "discomfort" sensation appears within the comfort region includes, for example, a case where multiple target persons (T) having different thermal sensations are in the indoor space (I) In such a case, a PMV with which all the target persons (T) feel comfortable can be set as the target PMV. In other words, even when there are multiple target persons (T) in the indoor space (I), thermal environment suitable for all the target persons (T) can be realized.

(8-6)
The control unit (100) of the embodiment employs, as a target PMV, the PMV with which the power consumption of the air conditioner (10) in operation is minimized in the comfort region.

This makes it possible to achieve both the comfort of the indoor space (I) and the energy saving of the air conditioner (10).

(8-7)
The control unit (100) of the embodiment sets the thermal index value between two comfort regions indicating comfort in the sensation region as a neutral thermal index value indicating thermal neutrality, and sets the target value based on the neutral thermal index value.

Here, the standard of the International Organization for Standardization (International Standard ISO 7730) recommends PMV = 0 as thermal neutrality (a thermal condition that the target person (T) feels neither hot nor cold) and -0.5 < PMV < +0.5 as comfort regions. However, there is a case where the first comfort region and the second comfort region of the output second distribution data do not satisfy -0.5 < PMV < +0.5 due to predetermined factors. Examples of the predetermined factors include a case where errors occur in detection values of sensors (the indoor temperature sensor (55) or the indoor humidity sensor (58)) that detect a thermal environment, and a case where a thermal sensation of the target person (T) is biased. In such a case, the output value of the PMV calculated by the control unit (100) does not match the thermal sensation of the target person (T).

In this embodiment, the second operating mode is selected in the comfort operation, so that the thermal index value (PMV) between the first comfort region and the second comfort region appearing in the output second distribution data is set as the neutral thermal index value (neutral thermal index value) with which the target person (T) feels neither cold nor hot. This allows the output value of the PMV calculated by the control unit (100) to match the thermal sensation of the target person (T).

In addition, even if the detection values of various sensors have errors, calibration of the sensors can be eliminated, leading to cost reduction.

In addition, since the PMV is corrected based on the distribution of the appearing sensation, it is possible to realize the air-conditioning control that always matches the thermal sensation of the target person (T).

(8-8)
The control unit (100) of this embodiment determines the duration of the comfort sensation, and excludes PMV where the duration is shorter than the predetermined time, thereby setting the first comfort region and the second comfort region.

In this manner, the thermal index value corresponding to the sensation which disappears in a relatively short time among the sensations exhibited by the target person (T) in the thermal environment is excluded. By removing such noise, the reliability of the first target PMV and the second target PMV can be improved.

(8-9)
In this embodiment, the thermal index value is a predicted mean vote (PMV), and the control unit (100) sets the neutral thermal index value, as PMV = 0, between two comfort regions.

In the present embodiment, the comfort region during the cooling operation is defined as a first comfort region, and the comfort region during the heating operation is defined as a second comfort region. Since the thermal environment between the first comfort region and the second comfort region is thermally neutral, such a position is corrected to PMV = 0 to easily correct the PMV to an appropriate PMV corresponding to each sensation that has appeared.

(8-10)
The control unit (100) of this embodiment sets two discomfort regions indicating discomfort in the sensation region, sets PMV = -0.5 to one of thermal index values between the comfort regions and the discomfort regions adjacent to each other, and PMV = +0.5 to the other.

Accordingly, the first comfort region and the second comfort region can be set in the range of -0.5 < PMV +0.5. In this manner, the PMV can be easily corrected to an appropriate PMV corresponding to each sensation that has appeared.

(8-11)
The control unit (100) of the embodiment controls the air conditioner (10) based on the target PMV.

Based on the first target PMV and the second target PMV, the indoor space (I) can be made into a thermal environment in which the target person (T) feels comfortable.

### (9) Variations

The above embodiments may be modified as follows. Differences from the above embodiments will be described below.

The control unit (100) of a variation transmits and receives predetermined information to and from an information terminal (80) including a predetermined display screen, such as a smartphone and a tablet. The information terminal (80) is an example of the communication device (80) of the present disclosure. The information terminal (80) is enabled to communicate with the control unit (100) by installing a predetermined application, and executes a predetermined program capable of instructing the control unit (100) to change the thermal index value.

The control unit (100) of the variation controls the air conditioner (10) based on the thermal index value set by the information terminal (80).

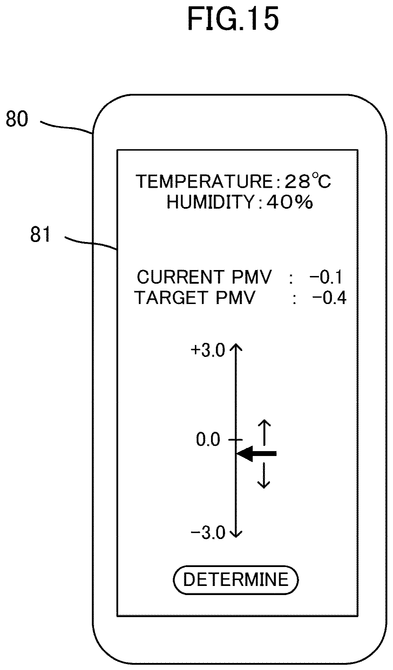

Specifically, the control unit (100) transmits the predetermined setting information including a target value of the thermal index to the information terminal. As illustrated in FIG. 15, the information terminal (80) which has received setting information displays an operation screen (81) on which an operation related to the setting of the thermal index value can be performed. As an example, the operation screen (81) displays information indicating the current thermal environment (the temperature and the humidity), the current thermal index value, the target value, and a setting screen for setting the thermal index value. The target value of the thermal index displayed here may be the first target PMV or second target PMV of the embodiment. Based on the displayed content, the target person (T) sets the thermal index value to a preferred thermal index value. The thermal index value after the setting is set as the set thermal index value. The operation screen may be a screen for adjusting the temperature, humidity, airflow direction, or the like. In this manner, the thermal index value can be changed indirectly.

The control unit (100) controls the air conditioner (10) based on the set thermal index value received by the information terminal. Specifically, the control unit (100) controls the air conditioner (10) so that the thermal index value for the indoor space (I) becomes the set thermal index value.

As described above, in the first embodiment, the target person (T) can grasp the target value by the information terminal (80). The target person (T) can control the thermal environment according to his/her preference based on the target value. This improves the convenience of the environment control device (E). The target person (T) can grasp the thermal state of the space (I) before and after the control, and thus can actually feel a change in thermal environment.

### (10) Other Embodiments

The above embodiments may be modified as follows.

### (10-1) Other Examples of Environment Adjustment Device

The environment adjustment device of the embodiments is an air conditioner (10) including an air conditioning portion (A). The environment adjustment device may be however another device as long as the device includes an environment adjustment portion capable of applying an environmental stimulus to the target person (T). The environment adjustment device may be, for example, a floor heating device, a bathtub water temperature adjustment device, a sauna device, or a sound generation device.

The floor heating device includes an environment adjustment portion that adjusts the temperature of the floor surface so as to apply a thermal stimulus to the target person (T). The bathtub water temperature adjustment device has an environment adjustment portion that adjusts the water temperature in the bathtub in which the target person (T) is present, so as to apply a thermal stimulus to the target person (T). The sauna device includes an environment adjustment portion that adjusts the temperature of a sauna space in which the target person (T) is present, so as to apply a thermal stimulus to the target person (T). The sound generation device includes an environment adjustment portion that emits sound, so as to apply a sound stimulus to the target person (T).

### (10-2) Other Examples of Infrared Ray Sensor

The infrared ray sensor (56) constitutes a location detector for identifying the locations of the target persons (T). The location detector may be, for example, an imaging device that captures still images or moving images of the target person (T). In this case, the imaging device may be a thermal camera.

### (10-3) Other Examples of Air Conditioner

The air conditioner (10) is a pair-type air conditioner including one indoor unit (30) and one outdoor unit (20). However, the air conditioner (10) may be of an indoor multi-type having two or more indoor units (30) or an outdoor multi-type having two or more outdoor units (20).

The air conditioner (10) may be a ventilator that provides air ventilation, an air purifier that purifies air, or a humidity control apparatus that humidifies or dehumidifies air. In other words, the "air conditioning" described herein means not only temperature control of air, but also ventilation of air, purification of air, and humidity control of air.

### (10-4) Examples of Thermal Index

The thermal index is not limited to PMV. The thermal index may be standard new effective temperature (SET), temperature-humidity index (THI, discomfort index), wet bulb globe temperature (WBGT), heat stress index (HSI), effective temperature (ET), and operative temperature (OT). The thermal index may be ambient temperature.

### (10-5) Other Examples of Environment Control Device

The environment control device (E) is not limited to the device of controlling the air conditioner (10) as long as including a control unit (100) used to control the air conditioner (10). For example, the environment control device (E) may be a device of outputting the target value of the thermal index or the information related to the target value, to the control unit (100) that controls the air conditioner (10). In this case, the environment control device (E) may be a cloud device wirelessly connected to the air conditioner (10) via a predetermined server, or may be a monitoring device that monitors the thermal environment of the indoor space (I). The monitoring device is connected to the server to which the air conditioner (10) and the cloud device are to be connected.

The environment control device (E) may be a communicative management device for the air conditioner (10). The management device is, for example, a server device, a centralized management device, or a terminal device. The terminal device may be a smartphone or a tablet terminal owned by the user.

The environment control device (E) may have a sensation estimation unit (not shown). The sensation estimation unit includes a radio-frequency sensor (57) and a predetermined arithmetic processing unit (not shown). The arithmetic processing unit performs arithmetic processing for estimating sensation based on the control unit biological signal. The sensation estimation unit may include another sensor instead of the radio-frequency sensor (57). The other sensor may be of contact type. The non-contact type sensor may be, for example, an acceleration sensor or electrocardiograph. The other sensor may be of non-contact type. The non-contact type sensor may be, for example, a tube-shaped pressure sensor or millimeter-wave radar.

The arithmetic processing unit of the sensation estimation unit may be configured as a separate element from the control unit (100). Specifically, for example, the sensation estimation unit may be configured as a unit including the sensor described above and the arithmetic processing unit and may output the sensation of the target person (T) estimated by the sensation estimation unit to the control unit (100). In this case, the control unit (100) controls the environment adjustment portion, based on the sensation of the target person (T) received from the unit.

### (10-6) Other Examples

The control unit (100) may include a storage unit (not shown). The storage unit stores first thermal data, second thermal data, first distribution data, and second distribution data.

If the control unit (100) associates the first target PMV or the second target PMV with the temporal change, the temporal change may be for each season or for each week. The control unit (100) may associate the first target PMV or the second target PMV with outdoor air temperature. In this case, the control unit (100) may set the first target PMV or the second target PMV for each certain outdoor air temperature range. The control unit (100) may associate the first target PMV or the second target PMV with the temporal change and the outdoor air temperature.

The control unit (100) may determine the first target PMV based on only the sensation data indicating "comfort" among the first thermal data. In this case, in Step S12, the discomfort region is not created.

The control unit (100) may determine the first target PMV based on only the sensation data indicating "discomfort" in the first thermal data. In this case, the first target PMV is determined based on the thermal index value where the appearance frequency of "discomfort" is equal to or lower than the predetermined value.

In Step S21, the control unit (100) may exclude, from the first data obtained in Step S12, the sensation data where the duration of "comfort" is shorter than the predetermined time, to set the comfort region. The control unit (100) may exclude, from the first data, the sensation data where the duration of "discomfort" is shorter than the predetermined time, to set the discomfort region.

In Step S21 or Step S32, the control unit (100) may not exclude the thermal index value in the comfort region in which the duration of comfort is shorter than the predetermined time.

In Step S21, the control unit (100) may not exclude the thermal index value in the discomfort region in which the duration of discomfort is shorter than the predetermined time when determining the first target PMV.

In Step S22, the control unit (100) may not exclude the thermal index value where the number of appearance of "comfort" is equal to or lower than the predetermined number of times.

In Step S23, the control unit (100) may not exclude, from the comfort region, the thermal index value where the discomfort sensation appeared.

In Step S25 or Step S34, the target person (T) may select the first target PMV or the second target PMV.

In the setting operation of the second target PMV, the corrected second distribution data (Step S33) may be directly outputted without outputting the second distribution data in Step S31.

In Step S33, either one of the correction of setting PMV = 0 between two comfort regions (the first comfort region and the second comfort region) or the correction of setting PMV = -0.5 to one of the thermal index values between the comfort region and the discomfort region adjacent to each other and setting PMV = +0.5 to the other may be performed.

The communication device (80) of first variation may be a remote controller (50).

The PMV may be calculated by at least one of the temperature or any of other conditions (humidity, airflow, radiation, amount of clothing, activity amount, etc.).

While the embodiments and variations thereof have been described above, it will be understood that various changes in form and details may be made without departing from the spirit and scope of the claims. The foregoing embodiments and variations thereof may be combined and replaced with each other without deteriorating the intended functions of the present disclosure. The expressions of "first," "second," . . . described above are used to distinguish the terms to which these expressions are given, and do not limit the number and order of the terms.

### INDUSTRIAL APPLICABILITY

As can be seen in the foregoing description, the present disclosure is useful for an environment control device, environment adjustment device, air conditioner, control method, and program.

### DESCRIPTION OF REFERENCE CHARACTERS

- 10: Air Conditioner (Environment Adjustment Device)
- 80: Communication Device (Information Terminal)
- 81: Operation Screen
- 100: Control Unit
- A: Air Conditioning Portion (Environment Adjustment Portion)
- E: Environment Control Device
- I: Indoor Space (Space)
- T: Target Person

## Claims

1. An environment control device comprising a control unit (100) used to control an environment adjustment portion (A) that controls a thermal environment of a space (I),
the control unit (100) being configured to
estimate a sensation indicating comfort or discomfort of a target person (T) based on sensation information about the target person (T) in the space (I),
determine, based on an appearance frequency of each sensation corresponding to a thermal index value as a value of thermal index, a range of the thermal index value indicating comfort or discomfort as a sensation region,
set a target value of the thermal index in the space (I) based on the sensation region,
determine a duration of comfort or discomfort sensation, and
determine the sensation region by excluding the thermal index value where the duration of the comfort or discomfort sensation is shorter than a predetermined time.

2. The environment control device of claim 1, wherein
the control unit (100) is further configured to associate the target value with a temporal change or an outdoor air temperature.

3. The environment control device of claim 1 or 2, wherein
the control unit (100) is further configured to
limit the sensation region to a comfort region in which the sensation indicates comfort, and
set the target value to a value in the comfort region.

4. The environment control device of claim 3, wherein
the control unit (100) is further configured to exclude, from the comfort region, one or more of the thermal index values where the sensation of discomfort appeared.

5. The environment control device of claim 3, wherein
the control unit (100) is further configured to set, to the target value, the thermal index value where power consumption of the environment adjustment portion (A) in operation is equal to or lower than a predetermined value in the comfort region.

6. An environment control device comprising a control unit (100) used to control an environment adjustment portion (A) that controls a thermal environment of a space (I),
the control unit (100) being configured to
estimate a sensation indicating comfort or discomfort of a target person (T) based on sensation information about the target person (T) in the space (I),
determine, based on an appearance frequency of each sensation corresponding to a thermal index value as a value of thermal index, a range of the thermal index value indicating comfort or discomfort as a sensation region,
set a target value of the thermal index in the space (I) based on the sensation region,
set the thermal index value between two comfort regions indicating comfort in the sensation region as a neutral thermal index value indicating thermal neutrality, and
determine the target value based on the neutral thermal index value.

7. The environment control device of claim 6, wherein
the control unit (100) is further configured to
determine a duration of the sensation of comfort, and
determine the two comfort regions by excluding the thermal index value where the duration is shorter than a predetermined time.

8. The environment control device of claim 6 or 7, wherein
the thermal index is a predicted mean vote (PMV), and
the control unit (100) is further configured to set the neutral thermal index value as PMV = 0 between the two comfort regions.

9. The environment control device of any one of claims 6 to 8, wherein
the thermal index is a predicted mean vote (PMV), and
the control unit (100) is further configured to
set two discomfort regions indicating discomfort in the sensation region, and
set PMV = -0.5 to one of the thermal index values between the comfort region and the discomfort region adjacent to each other, and set PMV = +0.5 to the other thermal index value.

10. The environment control device of any one of claims 1 to 9, wherein
the control unit (100) is further configured to control the environment adjustment portion (A) based on the target value.

11. The environment control device of any one of claims 1 to 10, wherein
the control unit (100) is
communicatively connected to a predetermined communication device (80) configured to display an operation screen (81) that allows setting of the thermal index value, and
configured to transmit setting information indicating the target value to the communication device (80) and receive information indicating the thermal index value set based on the setting information.

12. An environment adjustment device comprising the environment adjustment portion (A) and the control unit (100), of any one of claims 1 to 11.

13. An air conditioner comprising:
the environment adjustment portion (A) and the control unit (100), of any one of claims 1 to 12,
the environment adjustment portion (A) being an air conditioning portion (A) configured to condition air in the space (I).

14. A control method comprising:
estimating a sensation indicating comfort or discomfort of a target person (T) based on sensation information about a target person (T) in a space (I);
determining, based on an appearance frequency of each sensation corresponding to a thermal index value as a value of thermal index, a range of the thermal index value indicating comfort or discomfort as a sensation region; and
setting a target value of the thermal index in the space (I) based on the sensation region.

15. A program for causing a computer to execute:
estimating a sensation indicating comfort or discomfort of a target person (T) based on sensation information about a target person (T) in a space (I);
determining, based on an appearance frequency of each sensation corresponding to a thermal index value as a value of thermal index, a range of the thermal index value indicating comfort or discomfort as a sensation region; and
setting a target value of the thermal index in the space (I) based on the sensation region.
